# EUROPEAN PATENT SPECIFICATION

(11) **EP 3 562 495 B1**
(45) Date of publication and mention of the grant of the patent: **28.02.2024**
(21) Application number: 17842400.8
(22) Date of filing: 29.12.2017
(51) Int. Cl.: A61K 36/185, A61P 33/00, A61P 31/10, A61P 33/14, A01N 1/00, A01N 65/08, A01K 51/00

(54) **COMPOSITION FOR PREVENTION AND CONTROL OF BEE DISEASES AND USE OF HEMP OIL FOR PREPARATION OF THE COMPOSITION**
ZUSAMMENSETZUNG ZUR PRÄVENTION UND BEKÄMPFUNG VON BIENENERKRANKUNGEN UND VERWENDUNG VON HANFÖL ZUR HERSTELLUNG DER ZUSAMMENSETZUNG
COMPOSITION POUR LA PRÉVENTION ET LA LUTTE CONTRE DES MALADIES D'ABEILLES ET UTILISATION D'HUILE DE CHANVRE POUR LA PRÉPARATION DE LA COMPOSITION

(30) Priority: 29.12.2016 PL 42003616
(43) Date of publication of application: 06.11.2019
(73) Proprietor: Beemmunity Unlimited SP. Z O.O., 35-206 Rzeszów (PL)
(72) Inventor: KANIEWSKI, Ryszard, 60-665 Poznan (PL); BAJAS, Daniel, 60-665 Poznan (PL); MODLINSKI, Rafa, 60-665 Poznan (PL)
(74) Representative: Twardowska-Czerwinska, Aleksandra
(86) International application number: PCT/PL2017/000135
(87) International publication number: WO 2018/124896

(56) References cited:
- DATABASE WPI Week 201323 Thomson Scientific, London, GB; AN 2013-A39823 XP002779632, & PL 394 918 A1 (INST OCHRONY ROSLIN-PANSTWOWY INST BADAW) 19 November 2012 (2012-11-19)
- JOHANNES NOVAK ET AL: "Essential oils of different cultivars ofCannabis sativa L. and their antimicrobial activity", FLAVOUR AND FRAGRANCE JOURNAL., vol. 16, no. 4, 1 July 2001 (2001-07-01), pages 259-262, XP055463351, GB ISSN: 0882-5734, DOI: 10.1002/ffj.993

## Description

The subject of the invention is a composition for prevention and control of *varroosis.* In more detail, the invention relates to a composition comprising hemp essential oil for bee diseases prevention and control, especially parasitic diseases, including varroosis.

There are a lot of mites species that invade bee colonies. Most of them occupy the bottom of beehives and feed on the remains produced by the bees. However, few mites species lead parasitic life and may cause death of the entire bee colony.

Varroosis is a parasitic disease affecting adult bees as well as the brood. Only the Korean (Russian) type of mites *Varroa destructor* resides in Europe. Males of *Varroa destructor* reside only on capped brood or shortly after the bee emerges from her cell. Female mites occur only on adult bees. Mouthpart of *Varroa destructor* is of piercing and sucking type, adapted for acquiring haemolymph, which is the only feed for this parasite. The whole life cycle takes place in the capped brood, worker one as well as the drone brood. The female mites penetrates the brood cell right before its capping. Then, it lies few eggs, from 2 to 5, on the cell's wall. The first laid egg is unfertilized and after a few sheddings a male develops. From the other eggs hatch females. The male dies after an intercourse. The male life cycle lasts around 6.2 days and female life cycle around 6.9 days. The life cycle of worker bee under capping lasts 12 days, drone's 14 days and therefore not all daughter female specimens of mites gain full adolescence and are fertilized. It is assumed that the average of daughter females in bee brood cell amounts to 2-3 and in drone brood cell it amounts to 3-4 daughter females. The mites females leave the cells along with the emerging bees and transfer to the adult bees on which they feed (usually for around 14 days) and then they penetrate next brood cells in order to start new life cycle.

Unfertilized young mites female also penetrates the cell and lies only one unfertilized egg. The male developed that way fertilizes during the intercourse his own mother, who in the next life cycle gains her own daughters [1].

Mites in bee colonies are usually controlled with pesticides, including e.g. apiwarol based on amitraz, fipronil, fluvalinate, coumaphos and others. Pharmacological and breeding methods allowing for reduction of mites population in bee colony are used in controlling *Varroa destructor.*

In Poland there is also available a biological method for controlling *Varroa destructor* in form of beneficial *Stratiolaelaps scimitus* mites, which are applied to the beehive with bees during spring and autumn. The mites are too small to eat *Varroa destructor,* but fighting them damages it such that life cycle cannot continue.

The scientific research have shown that Korean type of Varroa destructor very quickly develops resistance to acaricides, especially those from the group of synthetic pyrethroids. Therefore occurs lowering effectiveness of drugs based especially on fulvalinate. Varroa destructor is very resistant to lack of feed, without which it can last up to 9 days. The mites can survive on cells with dead brood for up to 30 days.

The beekeepers face the problem of varroosis every year. Contaminated bee colonies die in autumn without human help and it is enough if the spring contamination reaches the level of 10 mites. The basis of running the apiary is to carefully "clean" bee colonies of these parasites, so that reinvasion does not occur from the treated apiaries.

Varroa destructor causes inter alia:
- losses in bee colonies caused by *Varroa destructor,*
- death of bee colonies during winter time,
- increased overwintering losses
- poor spring development
- low honey yield
- sudden weakening of colonies after foraging
- development deformations. [1]

Johannes Novak et al. in the publication "Essential oils of different cultivars of Cannabis sativa L. and their microbial activity" describes the use of hemp essential oil for its antimicrobial activities [6].

The PL217334 (publ. 2013-09-02) discloses use of essential oils mixture, more specifically, tea tree oil mixed with basil oil and/or fir oil for preparation of a formulation for controlling the mites in bee colonies, wherein mites include *Varroa destructor* or *Typhlodromus pyri Sch.*

PL186883 patent description (publ. 1997-12-18) discloses gel formulation with slow release and a method of controlling mites, butterflies, fungi and bacteria invasions on honey bee colonies, which comprises as an active substance essential oil or organic acid in concentration from about 5% to about 50%, preferably from 10% to 40% of whole formulation weight, thickeners with linking agent in concentration from 0.01% to 1.5%, preferably from 0.1% to 1.0% of whole formulation weight, carrier and water. The slow release formulation of the invention is effective against *Varroa jacobsoni* invasions of bee colonies when compared with control colonies.

A formulation based on hemp oil for pest control, especially true bugs, and preferably aphids is known from PL214762 (publ. 2011-03-28). A concentrate for preparation of aqueous emulsion based on hemp oil is meant for apple aphid (*Aphis pomi* Deg.) control and rosy apple aphid (*Dysaphis plantaginea* Pass.).

Additionally, PL394918 application (publ. 2012-11-19) describes a composition against pests, especially spider mites, based on hemp essential oil for pest control, mostly spider mites. Concentrate for preparation of hemp oil emulsion is intended mostly for red spider mite (*Tetranychus urticae* Koch) and carmine spider mite (*Tetranychus cinnabarinus* Bois.).

Although, a use of a composition based on hemp essential oil for control of spider mites is known, the attention should be drawn to the fact that there is around 30,000 species of mites, adapted for different environments, which results in species differentiation. The only common feature of mites is the conjunction of cephalothorax and abdomen and lack of segmentation signs on the outside. The use of the composition based on hemp essential oil against a particular mites species that are invading bees is not known in the prior art nor it is even suggested.

Additionally, although there are existing solutions in the prior art there is constant need for acquiring a composition for prevention and control of mites invading bees, especially that the previous solutions seem insufficient. The mites gain resistance to acaricides. The pesticides weaken the immune system of many animals and plants. Chemical formulations influence the contamination of honey and wax. Use of organic acids, such as formic acid, lactic acid or oxalic acid, on the other hand does not result in mites gaining the resistance to these acids, but is work consuming and dependent on atmospheric conditions and on bee colony development stadium and usually they are not sufficient. Anti-varroa formulations cannot be used during the marketed honey production period. Additionally, it is necessary to strictly obey grace periods required for specific formulations.

The aim of the invention is to control V. destructor with use of essential oil made of cannabis sativa (*Cannabis sativa* L.). These formulations are either repellants or toxic. It is important that the ingredients of the oil have significant impact on *Varroa destructor* control and may become a good alternative for chemical formulations.

The species from which extracts or oils could be used for bee protection from mites is cannabis sativa (*Cannabis sativa* L.). These species belong to Cannabis genus, Cannabaceae family. There are three species within the Cannabis genus, which are Cannabis indica (*Cannabis indica* Lam.), cannabis sativa (*Cannabis sativa* L.) and cannabis ruderalis (*Cannabis ruderalis* Janisch.).

The species of Cannabis genus contain narcotic substance called tetrahydrocannabinol (Δ⁹ THC). Due to the amount of this compound there are hashish (5 to 20% of THC) and fibrous (below 0.2% of THC) cannabis. The patent utilizes only the hemp essential oils produced from polish species of cannabis: Beniko and Bialobrzeska of low cannabinoid percentage (below 0.2% of dry hemp weight). Said species fulfill the requirements determined by the law (dated September 6, 2001, Journals of Law No. 125, pos. 1367) and are authorized for farming in Poland. In fact, the average amount of THC in oils in last 3 years equals to about 0.0237%.

The hemp essential oil has been described in detail in the patent application P-394918. It is transparent, bright yellow to dark green liquid. The relative density in the temperature of 20°C equals to about 0.840 g/cm³. The hemp oil comprises of about 58 monoteprenes and 38 sequiterpenes. The most important of them all are: α-pinene, β-pinene, Δ 3-carene, β-myrcene, limonene, β-phellandrene, cis-ocimene, trans-ocimene, α-terpinene, trans-α-bergamotene, β-caryophyllene, β-humulene, β-farnesene, β-selinene, selina-3,7(11)-diene.

The oil is obtained via water steam distillation of inflorescences (hemp straw) and young leaves. Due to characteristic aroma the oil is used for manufacturing different articles among which are for example cosmetics, food articles including alcoholic beverages. Additionally, the hemp oil is used also in aromatherapy and as pesticide, because two compounds of the hemp oil, limonene and α-pinene, repel the insects with their aroma.

A research has been conducted in respect of the use of hemp alone and its extracts as a repellant against different pests. Additionally, the hemp essential oil has bacteriostatic properties against Gram+ bacteria (*Staphylococcus, Streptococcus, Bacillus cereus, Bacillus subtilis, Staphylococcus aureus*) and Gram- bacteria *(Escherichia coli, Pseudomonas aeruginosa, Salmonella paratyphi, Shigella species).*

Surprisingly, it was discovered that the hemp essential oil may be used for prevention and control of bee diseases, especially parasitic diseases, including varroosis.

The subject matter of invention is a composition for prevention and control of *varroosis,* characterized in that it comprises hemp essential oil in concentration from 0.1 to 50%.

Preferably, the composition besides the hemp essential oil comprises natural solvent, including oils such as, but not limited to, essential oils, wax, propolis, ethanol extract of propolis (EEP), water, kerosene, alcohol, glycerin.

Preferably, the composition additionally comprises sunflower oil, wherein the hemp essential oil and sunflower oil ratio equals to 1:20.

Preferably, the composition is applied to bees using standard methods selected from smoking with oil using smoker devices, natural evaporation of oil from a small container placed in beehive, from wax, propolis or ethanol extract (EEP), infiltration of wooden matrix with oil and placing them in the beehive, sprinkling the bee spaces (free space between honeycombs in the beehive) with oil where the bees are located.

Preferably, the composition is applied to bees using standard methods selected from smoking with oil using smoker devices, natural evaporation of oil from a small container placed in beehive, from wax, propolis or ethanol extract (EEP), infiltration of wooden matrix with oil and placing them in the beehive, sprinkling the bee spaces (free space between honeycombs in the beehive) with oil where the bees are located. For better understanding of the invention example embodiments are shown below.

### Example 1

The hemp oil was used in 5% concentration by dissolving it in natural sunflower oil. The research was conducted on 10 bee colonies of Kwainka CTA6 species/line in "Varsovian" beehive type, of which 5 were test group, where the subject of the invention was used and the remaining were control group. An initial damage to bee colony by *V. destructor* was assessed before the research.

Initial damage to bee colony by *V. destructor*

| ***Test group*** | | | |
|---|---|---|---|
| Beehive no. | | *V. destructor* amount | |
| | 1 | | 13 |
| | 2 | | 17 |
| | 3 | | 15 |
| | 4 | | 8 |
| | 5 | | 10 |
| | Total: | | 63 |

| ***Control group*** | | | |
|---|---|---|---|
| | Beehive no. | *V. destructor* amount | |
| | 6 | | 25 |
| | 7 | | 12 |
| | 8 | | 11 |
| | 9 | | 16 |
| | 10 | | 17 |
| | Total: | | 81 |

Wooden strips infiltrated with hemp oil were placed between honeycombs in order to control *V. destructor* in beehives in the test group. Three stripes were used in each beehive. After 48 hours lethality of the parasite was assessed. Apiwarol in the amount of 12.5 mg was used in the control group as a comparison compound. The treatment was performed in two weekly repetitions (every seven days) directly on the bee colonies in the apiary.

Lethality of *V. destructor* parasite

| | *V. destructor* parasite deaths (pcs.) | |
|---|---|---|
| | 5% hemp oil | Apiwarol 12,5 mg |
| First treatment | 50 | 54 |
| Second treatment | 28 | 17 |

After 48 hours from the treatment, 5% hemp oil showed similar effectiveness in comparison with the Apiwarol formulation. During the second treatment, applied after seven days, a 60% better effectiveness has been noted for hemp oil when compared with the Apiwarol anti-varroa formulation.

### Example 2

The hemp oil was used in 0.5, 1 and 2.5% concentration by dissolving it in purified kerosene. The research was conducted on three selected bee colonies of Kwainka CTA6 species/line in "Great Poland" hive type with hygienic bottom. The treatment was conducted in two repeats with 3 days interval. After the research for determining the effectiveness of the subject of the invention, the control formulation of Apiwarol was used after 7 days.

### Example 2.1

0.5 ml of hemp oil was dissolved in 99.5 ml of purified kerosene to yield 0.5% concentration. 0.5% solution was evaporated in the beehive using electronic device in order to control *V. destructor* in bee colony. The lethality of the parasite was assessed 12 hours after treatment.

### Example 2.2

1 ml of hemp oil was dissolved in 99 ml of purified kerosene to yield 1% concentration. 1% solution was evaporated in the beehive using electronic device in order to control *V. destructor* in bee colony. The lethality of the parasite was assessed 12 hours after treatment.

### Example 2.3

2,5 ml of hemp oil was dissolved in 97,5 ml of purified kerosene to yield 2,5% concentration. 2,5% solution was evaporated in the beehive using electronic device in order to control *V. destructor* in bee colony. The lethality of the parasite was assessed 12 hours after treatment.

The effectiveness of the hemp oil in control of parasitic bee diseases including *V*. *destructor* was confirmed after conducting this research.

Lethality of the parasite after use of the hemp oil

| | *V. destructor* parasite deaths (pcs.) | |
|---|---|---|
| | Treatment 1 | Treatment 2 |
| Beehive no. 1 hemp oil - 0,5% | 18 | 11 |
| Beehive no. 2 hemp oil - 1% | 38 | 13 |
| Beehive no. 3 hemp oil - 2,5% | 28 | 7 |

Lethality of the parasite after the use of control formulation of Apiwarol after seven days from the use of hemp oil.

| | *V. destructor* parasite deaths (pcs.) |
|---|---|
| Beehive no. 1 | 6 |
| Beehive no. 2 | 7 |
| Beehive no. 3 | 3 |

The quantity of *V. destructor* parasite after the second treatment with evaporation of the hemp oil in the beehive has lowered by:
- 39% for the 0.5% concentration
- 64% for the 1% concentration
- 75% for the 2.5% concentration

After the control treatment with comparison formulation of Apiwarol, it was ascertained that the hemp oil has significant effectiveness. Deaths of *V. destructor* after the use of control test has lowered in average by 75% when compared to deaths determined after the first treatment.

### Example 3

The hemp oil was used in 50% concentration by dissolving it in purified kerosene. The research was conducted on 10 bee colonies of Kwainka CTA6 species/line in "Varsovian" beehive type of which 5 were test group where the subject of the invention was used and the remaining were the control group. An initial damage to bee colony by *V. destructor was* assessed before the research.

Initial damage to bee colony by *V. destructor*

| ***Test group*** | |
|---|---|
| Beehive no. | *V. destructor* parasite deaths (pcs.) |
| 1 | 27 |
| 2 | 15 |
| 3 | 33 |
| 4 | 26 |
| 5 | 19 |
| Total: | 120 |

| ***Control group*** | |
|---|---|
| nr ula | *V. destructor* parasite deaths (pcs.) |
| 6 | 22 |
| 7 | 17 |
| 8 | 31 |
| 9 | 11 |
| 10 | 24 |
| Total: | 105 |

A container filled with 50% hemp oil was placed in the edges of beehives in order to control *V. destructor* in beehives of the test group. A floral foam was placed in the containers to ease the evaporation of the formulation. Lethality of the parasite was assessed after 120 hours. 12.5 mg of Apiwarol was used in the control group as the comparison formulation.

Lethality of *V. destructor* parasite

| | 50% hemp oil | 12.5 mg Apiwarol |
|---|---|---|
| *V. destructor* parasite deaths (pcs.) | 41 | 69 |

After 120 hours from the treatment, 50% hemp oil has shown 59% effectiveness of hemp oil when compared with the comparison formulation of Apiwarol.

### References:

1. Pawet Chorbiński "Zapobieganie i zwalczanie chor6b pszczót"
2. Krystyna Pohorecka "Zwalczanie warrozy - propozycja modelu post 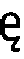 powania w warunkach polskich" Zaktad Parazytologii i Chor6b Inwazyjnych, PIWet-PIB w Pu 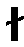 awach
3. Pawe 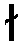 Chorbiński (2008) "Pokonaj warroz 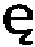 "
4. Maksymiuk W., Konopacka Z., (1986) "Gospodarka pasieczna na terenach zagrożonych warroz 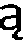 ", WOPR Halasy z/s w Grabanowie.
5. Cybulski W., Dzierżanowski A., (2010) "Analiza statystyczna wyników skuteczności APIWAROLU AS w badaniach prowadzonych w latach 1981-2009"
6. Johannes Novak et al. "Essential oils of different cultivars of Cannabis sativa L. and their microbial activity", Flavour and fragrance Journal, vol. 16, no. 4, 2001-07-01, p. 259-262, ISSN: 0882-5734.

## Claims

1. A composition for use in the prevention and control of *varroosis,* **characterized in that** it comprises hemp essential oil in concentration from 0.1 to 50%.

2. A composition for use according to claim 1, wherein the composition besides the hemp essential oil comprises natural solvent, including oils such as essential oils, wax, propolis, ethanol extract of propolis (EEP), water, kerosene, alcohol, glycerin.

3. A composition for use according to claim 1, wherein the composition additionally comprises sunflower oil, wherein the hemp essential oil and sunflower oil ratio equals to 1:20.

4. A composition for use according to claim 1, wherein the composition is applied to bees using standard methods selected from smoking with oil using smoker devices, natural evaporation of oil from a small container placed in beehive, from wax, propolis or ethanol extract (EEP), infiltration of wooden matrix with oil and placing them in the beehive, sprinkling the bee spaces (free space between honeycombs in the beehive) with oil where the bees are located.

## Patentansprüche

1. Zusammensetzung zur Verwendung bei der Prävention und Bekämpfung von Varroose, **dadurch gekennzeichnet, dass** sie ätherisches Hanföl in einer Konzentration von 0,1 bis 50% enthält.

2. Zusammensetzung zur Verwendung nach Anspruch 1, wobei die Zusammensetzung zusätzlich zu dem ätherischen Hanföl ein natürliches Lösungsmittel umfasst, das Öle einschließlich ätherischer Öle, Wachs, Propolis, ethanolischen Propolisextrakt EEP, Wasser, Paraffin, Alkohol, Glycerin umfasst.

3. Zusammensetzung zur Verwendung nach Anspruch 1, wobei die Zusammensetzung ferner Sonnenblumenöl enthält, wobei das Verhältnis von ätherischem Hanföl zu Sonnenblumenöl 1:20 beträgt.

4. Zusammensetzung zur Verwendung nach Anspruch 1, wobei die Zusammensetzung auf die Bienen unter Verwendung von Standardverfahren aufgebracht wird, die ausgewählt sind aus der Begasung mit dem Öl unter Verwendung von Verbrennungsvorrichtungen, der natürlichen Verdampfung des Öls aus einem kleinen Behälter, der in den Bienenstöcken platziert ist, aus Wachs, Propolis oder Ethanolextrakt (EEP), dem Tränken von Holzformen mit dem Öl und dem Platzieren derselben im Bienenstock, dem Besprühen der Bienenräume (freie Räume zwischen den Waben im Bienenstock) mit dem Öl, wo sich die Bienen befinden.

## Revendications

1. Composition destinée à la prévention et au contrôle de la *varroosis* (la varroose), **caractérisée par le fait qu'**elle contient de l'huile essentielle de chanvre à une concentration de 0,1 à 50 %.

2. Composition destinée à être utilisée selon la revendication 1, dans laquelle la composition comprend, en plus de l'huile essentielle de chanvre, un solvant naturel comprenant des huiles dont des huiles essentielles, de la cire, de la propolis, de l'extrait éthanolique de propolis EEP, de l'eau, de la paraffine, de l'alcool, de la glycérine.

3. Composition à utiliser selon la revendication 1, dans laquelle la composition comprend en outre de l'huile de tournesol, le rapport entre l'huile essentielle de chanvre et l'huile de tournesol étant de 1:20.

4. Composition destinée à être utilisée selon la revendication 1, dans laquelle la composition est appliquée aux abeilles par des méthodes standard choisies parmi la fumigation avec de l'huile à l'aide de dispositifs de combustion, l'évaporation naturelle de l'huile placé dans un petit récipient disposé dans les ruches, à partir de cire, de propolis ou d'extrait d'éthanol (EEP), l'imbibition de formes en bois avec l'huile et leur placement dans la ruche, la pulvérisation de l'huile dans des espaces réservés aux abeilles (espaces vides entre les rayons dans la ruche), dans les endroits où il y a des abeilles.
